# EUROPÄISCHE PATENTANMELDUNG

(11) **EP 2 520 597 A1**
(43) Veröffentlichungstag der Anmeldung: **07.11.2012**
(21) Anmeldenummer: 11164899.4
(22) Anmeldetag: 05.05.2011
(51) Int. Cl.: C08G 18/02, C07C 267/00, C08K 5/29

(54) **Carbodiimide aus trisubstituierten aromatischen Isocyanaten, ein Verfahren zu deren Herstellung und deren Verwendung**

(71) Anmelder: Rhein Chemie Rheinau GmbH, 68219 Mannheim (DE)
(72) Erfinder: Dr. Laufer, Wilhelm, 67158, Ellerstadt (DE); Dr. Bechem, Benjamin, 68157, Mannheim (DE); Dr. Eckert, Armin, 68794, Oberhausen (DE)
(74) Vertreter: Siegers, Britta

(57) **Zusammenfassung**

Die Erfindung betrifft neuartige Carbodiimide, ein Verfahren zu deren Herstellung und deren Verwendung als Stabilisator, Vernetzer und/oder Kompatibilisator in Thermoplasten, esterbasierten Polyolen für Polyurethan-Anwendungen, in Hartschaum, in Weichschaum oder z.B. in CASE-Anwendungen (Coatings Adhesives Sealants Elastomers).

## Beschreibung

Die Erfindung betrifft neuartige Carbodiimide, ein Verfahren zu deren Herstellung und deren Verwendung als Stabilisator, Vernetzer und/oder Kompatibilisator in Thermoplasten, esterbasierten Polyolen für Polyurethan-Anwendungen, in Hartschaum, in Weichschaum oder z.B. in CASE-Anwendungen (Coatings Adhesives Sealants Elastomers).

Carbodiimide haben sich in vielen Anwendungen bewährt, z.B. als Hydrolyseschutzmittel für Thermoplasten, Polyolen, Polyurethane etc.

Bevorzugt werden hierfür sterisch gehinderte Carbodiimide eingesetzt. Bekannt sind in diesem Zusammenhang vor allem das 2,6-Diisopropylphenyl-Carbodiimid (Stabaxol® I der Rhein Chemie Rheinau GmbH) und/oder Carbodiimide auf Basis 2,4,6-Triisopropylphenyl-1,3-diisocyanat (Stabaxol® P) oder auf Basis Tetramethylxylylendiisocyanat (Stabaxol® P 200). Die im Stand der Technik bekannten Carbodiimide haben jedoch die Nachteile in bestimmten Anwendungen, z. B. bei der Folienherstellung bei höheren Temperaturen, flüchtig oder thermisch nicht stabil zu sein und können flüchtige giftige Verbindungen abspalten und/oder müssen in der Regel aufgrund der niedrigeren Funktionalität unwirtschaftlich in hohen Mengen dosiert werden.

Es bestand daher ein Bedarf an Carbodiimiden, welche die vorgenannten Nachteile nicht aufweisen.

Aufgabe der vorliegenden Erfindung war daher die Bereitstellung von Carbodiimiden, welche temperaturstabil und wirtschaftlich herstellbar sind.

Überraschenderweise konnte diese Aufgabe durch bestimmte Carbodiimide gelöst werden.

Gegenstand der vorliegenden Erfindung sind daher Carbodiimide der Formel (I) 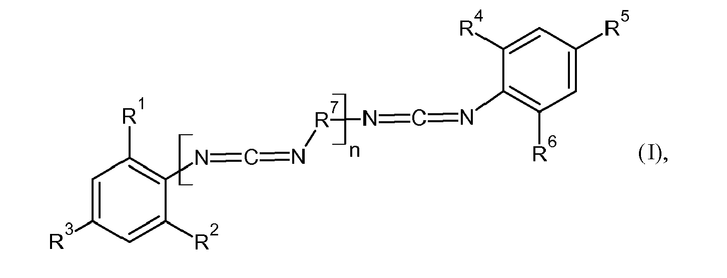 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₆-C₁₅-Aralkyl ist,
R⁷ = C₁ - C₁₈-Alkylen, C₅-C₁₈-Cycloalkylen-, Arylen und/oder C₇-C₁₈- Aralkylen und n eine ganze Zahl von 1 bis 500 ist. Vorzugsweise liegt n zwischen 1 und 50.

Die Alkylreste sind vorzugsweise verzweigt. In einer Ausführungsform der Erfindung sind die C₃-C₂₀-Alkylreste dabei linear und/oder verzweigt.

Bei den erfindungsgemäßen Carbodiimiden der Formel (I) sind die Reste R¹ bis R⁶ vorzugsweise gleich.

In einer weiteren bevorzugten Ausführungsform der Erfindung entsprechen die Reste R¹ bis R⁶ Isopropyl.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die Verbindungen der Formel (I) sind thermisch stabil und zeichnen sich durch eine hervorragende Wirksamkeit als Hydrolyseschutzmittel/Säurefänger in estergruppen-haltigen Polymeren aus.

Gegenstand der vorliegenden Erfindung ist zudem ein Verfahren zur Herstellung der erfindungsgemäßen Carbodiimide, wonach trisubstituierte Phenylisocyanate der Formel (II) 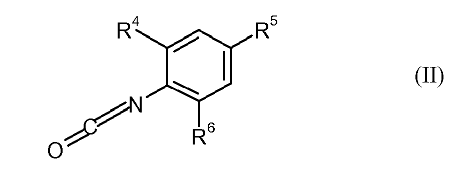 und der Formel (III) 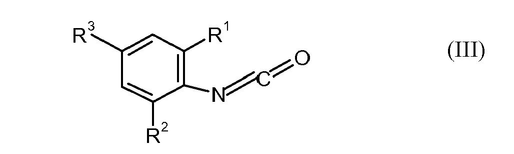 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₆-C₁₅-Aralkyl ist und Verbindungen der Formel (IV) III) 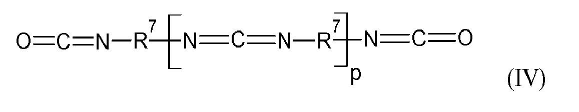 in der R⁷ = C₁-C₁₈-Alkylen, C₅-C₁₈-Cycloalkylen-, Arylen, C₇-C₁₈- Aralkylen ist und p ganze Zahlen von 0 - 500, bevorzugt 0 - 50 darstellt,
unter Abspaltung von Kohlendioxid bei Temperaturen von 40 °C bis 200 °C in Gegenwart von Katalysatoren und gegebenenfalls Lösungsmittel carbodiimidisiert werden.

Bei den trisubstituierten Phenylisocyanaten handelt es sich vorzugsweise um Isocyanate wie 2,4,6-Triisopropylphenylisocyanat, 2,6-Diisopropyl-4-*tert*-butylphenylisocyanat, 2,6-Diisopropyl-4-stearylphenylisocyanat, 2,6-Diisopropyl-4-dodecylphenylisocyanat, 2,4,6-Tri-*tert*-butyl-phenylisocyanat, 2,4,6-Tri-2-ethylhexylphenylisocyanat, 2,6-Diisopropyl-4-*n*-butylphenylisocyanat, 2,4,6-Tri-*n*-butylphenylisocyanat, 2,4,6-Tri-hexadecanylphenylisocyanat und/oder 2,4,6-Trioctadecanylphenylisocyanat.

Die trisubstituierten Phenylisocyanate können ausgehend von trisubstituierten Anilinen hergestellt werden.

Diese trisubstituierten Aniline können - wie dem Fachmann bekannt- über eine Friedel Crafts Alkylierung von Anilin mit dem entsprechenden Alken, Halogenalkan, Halogenalkenbenzol und/oder Halogencycloalkan hergestellt werden. Die Diarylanilin-Derivate können alternativ auch z.B. ausgehend von dem 2,6-Dibromanilin mittels Suzuki-Kupplung synthetisiert werden.

Anschließend werden diese trisubstituierten Aniline mit Phosgen zum entsprechenden trisubstituierten Phenylisocyanat umgesetzt. Die eingesetzten trisubstituierten Aniline sind auch kommerziell erhältlich, z.B. bei der Firma Lonza Group Ltd.

Bei den Verbindungen der Formel (IV) handelt es sich um handelsübliche Stoffe, die z.B. bei der Firma Rhein Chemie Rheinau GmbH z.B. unter dem Handelsnamen Stabaxol® P 220, Stabaxol® P 100 oder z. B. bei der Firma Bayer MaterialScience AG z.B. unter dem Handelsnamen Desmodur® W, Desmodur® I, Desmodur® 44 M und Desmodur® T erhältlich sind.

Die Carbodiimidisierung erfolgt dabei vorzugsweise gemäß der in Angew. Chem. 93, S. 855 - 866 (1981) oder DE-A-11 30 594 oder Tetrahedron Letters 48 (2007), S. 6002 - 6004 beschriebenen Verfahren.

Als Katalysatoren sind in einer Ausführungsform der Erfindung starke Basen oder Phosphorverbindungen bevorzugt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Alkali-, Erdalkalioxide oder -Hydroxide, - Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. In einer weiteren Ausführungsform der Erfindung wird die Carbodiimidisierung zuerst in Substanz und anschließend im Lösemittel durchgeführt. Als Lösemittel können z. B. Benzine, Benzol und/oder Alkylbenzole eingesetzt werden.

Die erhaltenen erfindungsgemäßen Carbodiimide können gegebenenfalls nach der Reaktion gereinigt werden. Die Reinigung der Rohprodukte kann mittels Umkristallisation erfolgen. Als geeignete Lösemittel für das Umkristallisieren können z. B. Alkylbenzole, Alkohole, Ketone, Ether oder Ester eingesetzt werden.

Gegenstand der vorliegenden Erfindung ist zudem die Verwendung der erfindungsgemäßen Carbodiimide als Stabilisator, Vernetzer und/oder Kompatibilisator in Thermoplasten, wie z.B. Polyethylenterephthalat (PET), Polybutylenterephthalat (PBT), Polytrimethylenterephthalat (PTT), in Thermoplastischen Polyurethanen (TPU), Copolyestern, wie das modifizierte Polyester aus Cyclohexandiol und Terephthalsäure (PCTA), in thermoplastischen Polyester Elastomeren (TPE E), Polymilchsäure (PLA) und/oder PLA-Derivaten, Polyhydroxyalkanoaten (PHA), stärkebasierten Kunststoffen, Polyamiden (PA), wie z.B. Polyamid 6, 6.6, 6.10, 612. 10, 11, 12, oder in Blends, wie z.B. PA/PET- oder PHA/PLA-Blends.

Gegenstand der Erfindung ist zudem die Verwendung der erfindungsgemäßen Carbodiimide als Hydrolyseschutzmittel in esterbasierten Polyolen, wie z.B. petrochemisch oder biobasierten Polyolen, für Polyurethan-Hart- und Weichschäume, estergruppen-enthaltende Polyurethanklebstoffe, Schmierstoffe und/oder Öle, wie z.B. in Transformatorenölen, und/oder für Polyurethan-basierte CASE (Coatings Adhesives Sealants Elastomers)- Anwendungen.

Gegenstand der vorliegenden Erfindung ist zudem die Verwendung der erfindungsgemäßen Carbodiimide in fester Form vorzugsweise zur Feststoffdosierung auf kontinuierlich arbeitenden Verarbeitungsmaschinen, wie z.B. Ein-, Zwei- und Mehrwellenextrudern, kontinuierlich arbeitenden Co-Knetern (Typ Buss) und diskontinuierlich arbeitenden Knetern, z.B Typ Banbury und anderen in der Polymerindustrie üblichen Aggregaten.

Gegenstand der vorliegenden Erfindung ist zudem die Verwendung der erfindungsgemäßen Carbodiimide bei der Herstellung von Kunststofffolien, insbesondere PET-Folien, TPU-Folien und PLA-Folien.

Die nachfolgenden Beispiele dienen der Erläuterung der Erfindung, ohne dabei limitierend zu wirken.

### Ausführungsbeispiele

Ein Carbodiimid auf Basis Tetramethylxyloldiisocyanat, umgesetzt mit Polyethylenglykolmonomethylether, erhältlich unter der Bezeichnung Stabaxol ® P 200 der Firma Rhein Chemie Rheinau GmbH im Vergleich zum erfindungsgemäßen Carbodiimid auf Basis Tetramethylxyloldiisocyanat, umgesetzt mit Triisopropylphenylisocyanat.

Die vorgenannten Carbodiimide wurden bezüglich der Hydrolyseschutz/Säurezahlabbau-Wirkung in esterbasierten Polymeren geprüft.

### Herstellung eines erfindungsgemäßen Carbodiimides

In einem ausgeheizten und mit Stickstoff befüllten 500 ml Planschliffkolben wurden unter Stickstoffstrom 400 g Carbodiimid auf Basis Tetramethylxylylendiisocyanat und 210 g 2,4,6-Triisopropylphenylisocyanat vorgelegt und auf 140 °C erwärmt. Nach Zugabe von 400 mg 1-Methyl-phospholenoxid wurde das Reaktionsgemisch innerhalb von 5 Stunden auf 180 °C erhitzt. Im Anschluss wurde bei 180 °C solange umgesetzt bis ein NCO-Gehalt von < 1 % erreicht worden ist.

### Testergebnisse

Die Testergebnisse mit den erfindungsgemäßen Carbodiimiden zeigen, dass diese im Vergleich zu den im Stand der Technik bekannten Carbodiimiden eine sehr hohe Wirksamkeit als Hydrolyseschutzmittel/Säurefänger in esterbasierten Polymeren und/oder in esterbasierten Formulierungen bei gleichzeitig verbesserter thermischer Stabilität aufweisen.

## Patentansprüche

1. Carbodiimide der Formel (I) 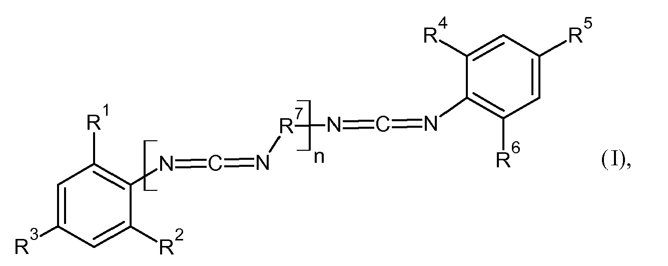 in der R¹, R², R³, R⁴, R⁵ und R⁶ unabhängig voneinander = C₁-C₂₀-Alkyl, C₃-C₂₀-Cycloalkyl, C₆-C₁₅-Aryl und/oder C₆-C₁₅-Aralkyl ist,
R⁷ = C₁-C₁₈-Alkylen, C₅-C₁₈-Cycloalkylen-, Arylen und/oder C₇-C₁₈- Aralkylen und
n eine ganze Zahl von 1 bis 500 ist.

2. Carbodiimide nach Anspruch 1, **dadurch gekennzeichnet, dass** die Reste R¹ bis R⁶ innerhalb des Moleküls gleich sind.

3. Carbodiimide nach Anspruch 1 oder 2, **dadurch gekennzeichnet, dass** die Reste R¹ bis R⁶ = Isopropyl sind.

4. Verfahren zur Herstellung der Carbodiimide nach einem oder mehreren der Ansprüche 1 bis 3, **dadurch gekennzeichnet, dass** trisubstituierte Phenylisocyanate der Formel (II) 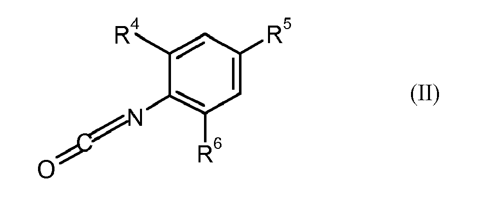 und der Formel (III) 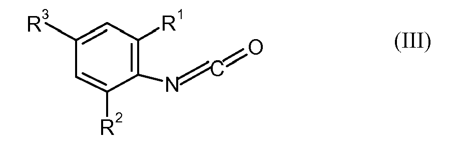 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₆-C₁₅-Aralkyl ist,
und Verbindungen der Formel (IV) 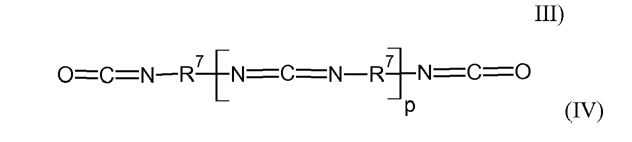 in der R⁷ = C₁ -C₁₈-Alkylen, C₅-C₁₈-Cycloalkylen-, Arylen, C₇-C₁₈- Aralkylen ist und p ganze Zahlen von 0 - 500, bevorzugt 0 - 50 darstellt,
unter Abspaltung von Kohlendioxid bei Temperaturen von 40 bis 200 °C in Gegenwart von Katalysatoren und gegebenenfalls eines Lösemittels carbodiimidisiert werden.

5. Verwendung der Carbodiimide nach einem oder mehreren der Ansprüche 1 bis 3 als Stabilisator, Vernetzer und/oder Kompatibilisator in Estergruppen enthaltenden Kunststoffen.

6. Verwendung der Carbodiimide nach einem oder mehreren der Ansprüche 1 bis 3 als Hydrolyseschutzmittel für esterbasierte Polyole, Polyurethan-Hart- und Weichschäume, estergruppen-enthaltende Polyurethanklebstoffe, Schmierstoffe und/oder Öle.

7. Verwendung der Carbodiimide nach einem oder mehreren der Ansprüche 1 bis 3 zur Feststoffdosierung auf kontinuierlich oder diskontinuierlich arbeitenden Verarbeitungsmaschinen.

8. Verwendung der Carbodiimide nach einem oder mehreren der Ansprüche 1 bis 3 zur Herstellung von Kunststofffolien, insbesondere PET-Folien, TPU-Folien und PLA-Folien.
